# EUROPEAN PATENT APPLICATION

(11) **EP 3 561 512 A1**
(43) Date of publication of application: **30.10.2019**
(21) Application number: 18168728.6
(22) Date of filing: 23.04.2018
(51) Int. Cl.: G01N 33/53, G01N 33/558, G01N 33/543

(54) **METHODS TO IMPROVE PERFORMANCE OF LATERAL FLOW TESTS**

(71) Applicant: Mak, Wing Cheung, 58331 Linkoping (SE); Filippini, Daniel, 58229 Linkoping (SE)
(72) Inventor: Mak, Wing Cheung, 58331 Linkoping (SE); Filippini, Daniel, 58229 Linkoping (SE)
(74) Representative: Gill Jennings & Every LLP

(57) **Abstract**

The present application is about a method to improve the analytical performance of lateral flow devices and extending their capabilities by allowing multiplexing configurations, customising test response and controlling the flow regime through the control of the membrane architecture using techniques compatible with the regular fabrication workflow. In a first aspect, an assay strip has a test zone of reduced cross-sectional area compared to the remainder of the assay strip. In a second aspect, an assay strip has a plurality of test zones through which a sample is successively drawn by capillary action. There is also described a method of producing the assay strips in which biorecognition molecules are applied to a sheet of membrane material, and the membrane material is then shaped into assay strips, each assay strip having a flow path including a test zone formed from a region of the strip to which biorecognition molecules have been applied.

## Description

### BACKGROUND

Lateral-flow tests, also known as lateral-flow immunochromatographic tests, have been available for many years. The simple urine-based human chorionic gonadotropin lateral flow tests remain a very popular analytical platform for home-based pregnancy tests. The design of the lateral-flow test is based on integration of various membrane components in a serial, lateral fashion with small overlaps connecting individual membranes, with each of the membranes able to perform a specific assay task.

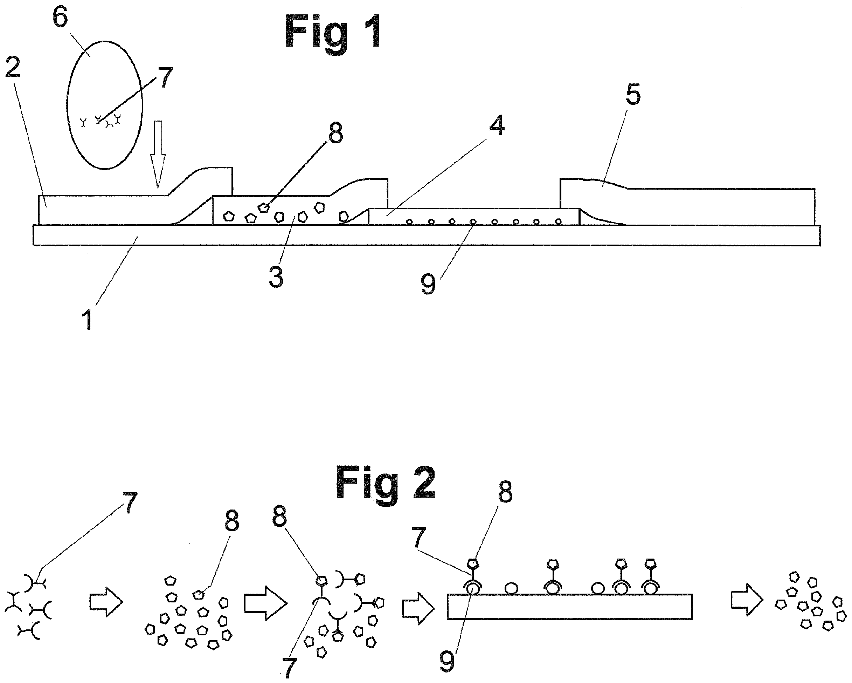

A schematic example illustrating a lateral-flow test is shown in Figure 1, while Figure 2 schematically illustrates the operation of the lateral-flow test.

Referring now to Figures 1 and 2, a lateral-flow test comprises a sample pad, a conjugation pad, a nitrocellulose membrane and an adsorption pad, all formed from porous or fibrous material through which liquid can flow. The lateral-flow device of Figure 1 includes a supporting substrate 1, on which are arranged the sample pad 2, the conjugation pad 3, the nitrocellulose membrane 4, and the absorption pad 5.

The sample pad 2, conjugation pad 3, membrane 4 and absorption pad 5 are in overlapping relationship so that a liquid sample placed on the sample pad 2 is first drawn into the sample pad 2 by capillary action to fill the sample pad 2, and is then drawn into the portion of the sample pad 2 overlapping the conjugation pad 3, from where it passes by capillary action into the conjugation pad 3, eventually filling the conjugation pad 3.

From the conjugation pad 3 the sample is then drawn by capillary action into the nitrocellulose membrane 4, and finally into the absorption pad 5. As the absorption pad 5 fills, the sample flows through the nitrocellulose membrane 4. The sample pad, conjugation pad, membrane and absorption pad may not be overlapping but may simply about each other, provided that liquid is able to flow by capillary action from one element to the next. The sample pad, conjugation pad, membrane and absorption pad may not be formed from the same porous material.

Typically, the sample pad, conjugation pad, and absorption pad may be formed from any suitable microporous material for lateral flow membranes. Such materials are known in the art and include nitrocellulose, nylon, cellulose acetate, PES (polyethersulfone), PVDF (polyvinylidenfiuoride), crosslinked dextran and other porous polymers. Preferably, the microporous membrane layer is a nitrocellulose layer. Nitrocellulose membrane layers for lateral flow assays are well-known in the art and are composed of interconnected nitrocellulose rods in a sponge-like structure.

The sample pad 2 is used to collect the sample fluid 6 containing the target analytes 7. In some cases the sample pad 2 is also able to perform sample pretreatment, such as separation of red-blood cells from the assay sample. The collected fluid is then drawn by capillary action into the conjugation pad 3, which is pre-loaded with biolabels 8 (mainly for visual or optical detection) that are specific for the target analytes 7 in the sample. The biolabels 8 attach to the target analytes 7 in the conjugation pad 3 as the sample fills and moves through the conjugation pad. The mixture is then drawn through the nitrocellulose membrane 4, which includes a test zone in which biorecognition molecules 9 are attached to the porous membrane. The biorecognition molecules 9 may be positioned in the test zone by applying to the test zone a quantity of liquid including the biorecognition molecules 9, and allowing the liquid to evaporate.

The affinity assays take place in the test zone, the target analytes 7, with their respective biolabel molecules 8 attached to them, also attach to the binding molecules 9, capturing the target analytes 7 in this zone. The sample fluid continues to flow through the test zone, bringing more analytes 7 to attach to the biorecognition molecules 9, and the fluid is collected by the adsorption pad 5. The rate at which the aqueous liquid flows through the porous membrane depends on the material and the porosity of the membrane.

The test result is obtained by visually or optically interrogating the test zone, where the captured analyte molecules with their bio labels produce an apparent colour change on the membrane.

Lateral-flow tests provide a simple, all-in-one platform for the rapid detection of analytes in a single step. The flexibility which results from combining various membrane components via a simple lamination process, makes lateral-flow technology an attractive and cost-effective analytical platform for applications in the areas of clinical diagnostics, food and water safety, pharmaceutical analysis and drug testing.

In conventional fabrication processes for shaped membranes, an elongate strip of substrate has applied to it lengthwise strips of absorbent materials which will form the sample pads, conjugation pads, membranes and absorption pads of a plurality of assay strips. Transverse cuts are then made through the absorbent materials in order to form cutaway portions which separate them into individual assay strips. There then follows a deposition process in which a solution of biorecognition molecules is applied across the separated membranes to form a test zone in each membrane. As the solution dries, biorecognition molecules are fixed in the membrane and form the test zone of the membrane.

Deposition of the solution of biorecognition molecules may be achieved by passing the membranes successively beneath a depositor nozzle to apply a "line" of the solution across the widths of successive separated membranes, keeping the depositor nozzle in contact with the membrane surface. As the nozzle passes over the separated membranes, the difference in height between the membranes and the cutaway portions of the membranes results in movement of the nozzle, which adversely affects the quality of the deposition process. Furthermore, some of the solution may be wasted by being deposited in cutaway areas of the substrate which are outside the membrane, and may be re-absorbed into the membrane, introducing contamination.

Paper sheets have been proposed to be used as capillary membranes, and in these paper-based devices flow control has been achieved by configuring each sheet with a two-dimensional layout where the liquid can flow. The stacking of two or more of these 2D layouts produces a thicker absorbent layer whose configuration may vary in the thickness dimension. This technology is known as "3D paper fluidics".

Paper analytical device layouts require a hydrophobic barrier to confine the flow to the desired layout, which is normally implemented using solid-ink (wax) printer and heating the printout to allow the waxy hydrophobic ink to permeate through the paper to create flow confinement barriers. However, the methods used in paper fluidics to define the flow layout require multiple chemical deposition/treatment steps and result in a pattern with poor resolution. This methodology is not compatible with nitrocellulose membranes and does not readily integrate into conventional lateral flow device manufacturing.

There are other physical techniques e.g. hand or computerized cutting, and laser ablation which have been used to create patterns on a paper sheet.

However, the above proposals were aimed at improving the accuracy of cutting paper or nitrocellulose capillary membranes. The disclosures do not address the problem of improving the analytical performance and signal acquisition for lateral flow tests.

Nitrocellulose membranes are preferred in commercial devices because of their superior efficiency to immobilize the binding chemistry, and their uniformly reproducible structure. The creation of a micro-structured fluidic pattern to control fluid flow in a nitrocellulose membrane remains a challenge.

Conventionally, the improvement of analytical performance of lateral flow tests mainly relies on the development of advanced biolabel systems such as gold nanoparticle labels, dye-doped latex particle labels, fluorescence labels, quantum dot labels and carbon-based nanoparticle-labels. The improved analytical performance is delivered because of enhanced signal output from the biolabel system. However, once the membrane porosity has been selected attending the flow rate requirements and demanded sample volume compatible with the detection chemistry there has not hitherto been room for further refinement of the device performance.

A first aspect of the present invention provides a qualitative assay device with improved sensitivity, and methods of manufacturing a capillary membrane for such an assay device. In the assay device according to this aspect of the invention, the cross-sectional area of the strip at the test zone is made smaller than the remainder of the strip, by reducing the width and/or thickness of the capillary membrane at the test zone.

The analyte molecules are thus constrained to pass through a smaller volume of the membrane where the biorecognition molecules are positioned at the test zone. The number of biolabelled analytes captured per unit volume of the test zone is increased. When biolabelled analyte molecules are captured by the biorecognition molecules, the bio labels are closer together and this increases the intensity of the colour change or light emission at the test zone, leading to a more sensitive assay because the more intense colour or light is more readily detectable, for example by the eye.

A second aspect of the invention addresses the problem of uneven deposition of the biorecognition molecules, by providing a method of manufacturing a membrane for an assay device with a shaped porous membrane in which the solution of biorecognition molecules is deposited in a linear region extending across an unshaped membrane blank, and subsequently the membrane blank is shaped by cutting or other suitable procedures to define a flow path for the sample liquid, the flow path being positioned on the membrane such that the test zone of the flow path coincides with a region of the membrane in which the biorecognition molecules have been deposited. The solution of biorecognition molecules may be deposited as a continuous line, or may be a series of discrete areas. The membrane is preferably shaped by a laser ablation process.

A third aspect of the invention provides a method for producing an assay strip comprising a flow path in which two or more test zones are formed, by depositing biorecognition material in a linear zone along a capillary membrane, and then forming the capillary membrane to define a flow path which passes through the linear zone a plurality of times.

The membrane may be formed from porous nitrocellulose, and may be modified chemically by the addition of functional groups which enable the nitrocellulose to couple to other molecules. Alternatively, or additionally, the nitrocellulose membrane may be modified to support cell life, for example by attaching cell nutrients to, or including nutrients in, the nitrocellulose fibres, the membrane 4 is normally cut into strips, for example about 5 mm wide and about 100 to 200 µm thick, to be integrated into a cartridge holder. The nitrocellulose membrane's porosity is chosen to produce a desired flow regime, and that limits the options to control the device's performance.

Typically, the nitrocellulose membrane has an open-pored structure. In addition, the capillary flow in the classical geometry imposes limitations to the minimum sample volume, which is necessary not only to contain a detectable amount of analyte, but also as a carrying fluid. Typically an assay strip is about 5 mm wide and about 0.2 to 0.5 mm thick, and the minimum sample volume required depends on the length of the membrane strip (i.e. the length of the flow path). Also, the sample pad for loading the sample solution will account for a significant dead volume requirement.

In a membrane having a constant width and thickness, a sample liquid initially enters the membrane at an upstream end with an initial flow rate. As the membrane is filled and the leading surface of the sample liquid moves along the membrane, the flow rate diminishes in a membrane of constant cross-section. The inventors have discovered that by controlling the variation of the cross-section of the membrane along its length in the flow direction, the rate of advance of the leading surface of the sample at particular points along the length of the amendment may be controlled, and this enables the flow of the entire liquid "column" advancing through the membrane to be controlled.

The flow rate may be controlled so as to be for example constant, or so as to have a predetermined flow rate followed by pause or a reduced flow for a predetermined time and followed by a resumption or increase of flow. By pausing or reducing the flow rate, the sample may be caused, for example, to be rapidly drawn through the conjugation zone where analyte molecules attach to biolabels, and then to dwell for a period at, or to flow more slowly through, a test zone in order to maximise the capture of labelled analytes by the biorecognition molecules positioned at the test zone.

The manufacturing method provides for the control of fluid flow through a capillary membrane by producing a specific 3D geometry in a nitrocellulose membrane by sculpting the membrane. The preferred technique for sculpting the membrane is laser ablation, but other suitable techniques may be used. The method allows for improved flow and performance control for lateral flow devices. The technique involves a fabrication step which is easily integrated with the large-scale manufacturing of nitrocellulose membrane lateral flow devices.

In an elongate porous membrane, a liquid sample placed at one end of the membrane is drawn by capillary action along the length of the membrane so that a "leading surface" of the liquid moves along the length of the membrane. If the membrane defines a flow path which is of a constant cross-sectional area, the speed of movement of the leading surface decreases as the surface moves along the membrane drawing a column of liquid of ever-increasing length. If the membrane is sculpted so as to define a test zone followed by a flow path for the liquid whose cross-sectional area gradually increases along the flow direction, a substantially constant speed of advance of the leading surface can be achieved, which leads to a flow regime in which the liquid passes through the test zone at a substantially constant rate. Conversely, if the flow path downstream of the test zone is arranged so as to have a decreasing cross-sectional area, a flow regime can be produced in which the liquid is drawn more slowly through the test zone.

Lateral flow device devices for qualitative tests typically report the test result directly visually to the user, by a line of colour appearing against a contrasting background when the target analyte is detected. This configuration is sufficient for many qualitative tests, such as pregnancy tests, where the test is to detect simply the presence or absence of the target analyte.

However, quantitative tests deliver a result dependent on the intensity of the colour or light appearing in the test zone, and for this they require a reader device with integrated illumination to quantify the intensity of the test line. Spurious ambient illumination can interfere with this detection.

This aspect of the present invention, is also applicable in a quantitative assay device including a capillary membrane in which a flow path sequentially passes through a plurality of test zones, each test zone comprising a number of biorecognition molecules which immobilise target analyte molecules. The quantity of biorecognition molecules at each test zone is predefined so that the total amount of target analyte captured at that test zone may be predicted. The test zones may each capture the same amount of target analyte, or may capture different amounts of target analyte. The amount of target analyte captured at each test zone may decrease or increase in the direction along the flow path.

The ability to define the membrane layout in this way enables the test zone to be embodied as a series of spaced test zones, with each test zone only reporting a positive signal when a defined analyte concentration has been reached at that location. As the analyte solution progresses along the flow path, the leading part of the analyte solution passes through successive test zones each of which removes a number of bio labelled analyte molecules from the solution. The strip may have an absorption pad of sufficient size that substantially all of the analyte is drawn through all of the test zones. Alternatively, the strip may be arranged so that only a leading portion of the analyte solution passes through all of the test zones.

A quantitative readout can be attained by visual or optical inspection to determine which zones report a positive signal and which do not. The reading is robust to ambient conditions since is the number and/or position of positively-reporting test zones that quantifies the response of the assay rather than the intensity of colour at any of the test zones.

Assay strips are known in which a predetermined volume of the sample is sequentially passed along a flow path through a number of test zones. Such strips are conventionally produced by depositing the biorecognition molecules in sequential depositions steps, or by using parallel nozzles to simultaneously deposit the biorecognition molecules of all of the test zones. A third aspect of the invention seeks to provide a novel structure and manufacturing method for producing assay strips having multiple test zones.

According to this aspect of the invention, a method for producing an assay strip having multiple test zones comprises depositing biorecognition material, in a single deposition step, in a linear zone extending along a capillary membrane supported on a substrate, and forming a flow path in the capillary membrane which crosses the linear zone at least twice, so that the intersections between the flow path and the linear zone form at least two test zones spaced along the length of the flow path.

Preferably the flow path is a serpentine flow path which crosses and re-crosses the linear zone a plurality of times. The biorecognition material may be deposited as a continuous strip in the linear zone, or may be deposited as a series of discrete regions within the linear zone, the flow path being arranged to cross the linear zone at these discrete regions. Because the biorecognition material is deposited onto the membrane before the membrane is shaped or separated into individual strips, a smooth depositions surface is available and even distribution of the biorecognition material along the linear zone is achieved, which leads to uniform amounts of biorecognition material in each of the test zones.

At the first test zone an amount of the target analyte may be captured which is sufficient to produce a positive result at that test zone. The sample then passes on to the next test zone with a reduced concentration of target analyte, and a further amount of target analyte is captured at the second test zone. The sample, with its further reduced concentration of target analyte then passes to the next test zone, and so on. Eventually, the sample arrives at a test zone at which the concentration of target analyte remaining in the sample is insufficient to produce a positive test result. The user may then simply visually inspect the assay device and count the number of test zones which have produced a positive result, in order to determine the concentration of target analyte in the original sample. The predetermined volume of the sample may be moved through the test zones by providing a large absorption pad which draws substantially all of the sample through the test zones. Alternatively, an inert liquid may be added after the test sample in order to drive the test sample through the test zones. As a further alternative, the absorption pad may have a predetermined capacity in order to ensure that a predetermined volume of the sample is drawn through the test zones before the absorption pad is filled and movement of the liquid ceases.

The assay device may be printed with additional indicia to assist the user in counting the test zones which return a positive result. Alternatively, the assay device may be printed with indicia which directly indicate a concentration of target analyte adjacent to each test zone, and the user may be directed to take the largest indicated concentration as the test result.

In some embodiments, two identical flow paths may be provided for the sample and may be fed by the same sample. In one flow path each test zone has a first number of biorecognition molecules, and in the other flow path each test zone may have a larger number of biorecognition molecules. As the sample passes through each test zone, the biorecognition molecules capture labelled analyte molecules, reducing the number of analyte molecules in the sample passing along that flow path. If the number of analyte molecules in the portion of the sample passing along the first flow path is such that every test zone captures a full load of analyte molecules, this only shows that the concentration of analyte in the sample is above a certain threshold. If the test zones in the second flow path each capture a portion of the labelled analytes in the part of the sample passing along second flow path such that one or more of the test zones at the downstream end of the flow path do not capture sufficient analytes to give a positive reading, then an accurate indication of the concentration of analytes in the sample can be determined. Essentially, each of the flow paths will detect a concentration of analytes in the sample which is within a specific range.

Embodiments of the invention will now be described in detail with reference to the accompanying drawings.

### BRIEF DESCRIPTION OF THE DRAWINGS

Figure 1 is a schematic side view of a conventional lateral flow assay device;
Figure 2 illustrates the stages in the assay test procedure;
Figure 3 is a perspective view of a test strip according to the first aspect of the present invention;
Figures 4A to 4F are schematic plan views of membranes for qualitative assay devices according to the first aspect of the present invention;
Figures 5A to 5F are top views of assay strips corresponding to the schematic views of Figures 4A to 4F;
Figure 6 is a plan view showing a number of test strips having different micro-fluidic concentrator geometries;
Figure 7 illustrates schematically a second test strip according to the first aspect of the present invention;
Figure 8 is a perspective view of a quantitative lateral flow test strip having a number of test zones;
Figure 9 is a schematic side view similar to Figure 1 of a test strip having a number of test zones;
Figure 10 illustrates the stages in the assay procedure of the device of Figure 9;
Figure 11 is a plan view of a second test strip according to the second aspect of the present invention;
Figure 12 is a perspective view of the capillary membrane of the test strip of Figure 11;
Figure 13 is a perspective view of a test strip comprising two serpentine flow paths arranged in parallel; and
Figure 14 is a perspective view of a test strip having a membrane in which three pairs of serpentine flow paths are fed from the same conjugation pad;
Figure 15 is a perspective view of a further embodiment of the test strip, in which the membrane has a pair of serpentine paths with their straight portions aligned along the length of the strip; and
Figures 16A to 16D are schematic views of steps in a manufacturing process for producing shaped membranes.

### DESCRIPTION

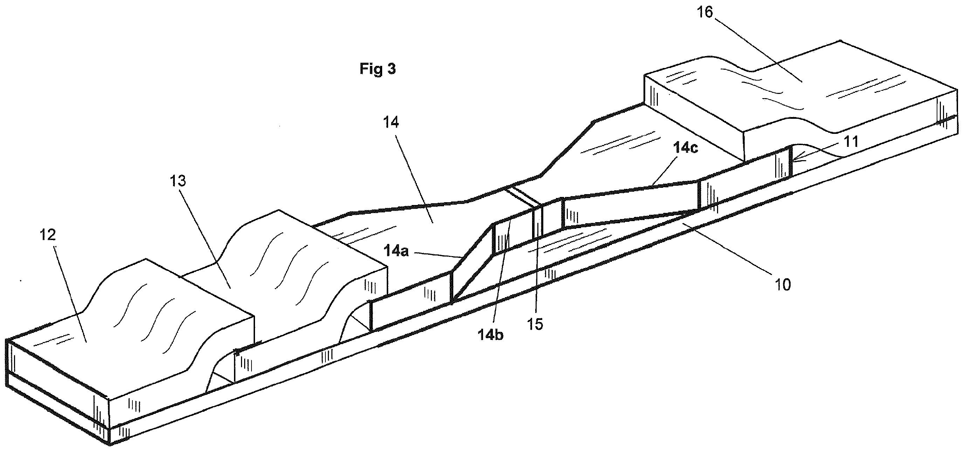

The first aspect of the invention provides a micro-fluidic concentrator for enhancing assay sensitivity, and is illustrated in Figures 3 to 10.

Referring now to Figure 3, the test strip comprises an impervious hydrophobic substrate 10, usually formed from a plastics material but possibly alternatively formed from a paper substrate coated with hydrophobic layer (e.g. wax or polymer). Exemplary materials for a plastics substrate are PMMA, polyethylene PET or similar materials.

The substrate 10 provides a support for a porous capillary layer 11 formed from nitrocellulose. The substrate 10 also provides support for a sample pad 12, corresponding to the sample pad 2 of the device of Figure 1, onto which the sample for analysis is deposited. The first zone 12 extends across the entire width of the supporting substrate 10, a distance of typically 5 mm.

The downstream edge of the sample pad 12 overlaps a conjugation pad 13 corresponding to the conjugation pad 3 of the device of Figure 1. The conjugation pad 13 itself overlaps the upstream end of the capillary membrane 14. The conjugation pad 13 is impregnated with appropriate biolabels for binding to the analyte whose presence is to be detected. The density of impregnation with biolabels in this zone may be constant, or may increase or decrease along the flow direction of the test liquid.

The downstream end of the conjugation pad 13 overlaps a nitrocellulose membrane 14. The membrane 14 includes a narrowing portion between tapered sides 14a, and a central narrowed region 14b including an indicator region 15. The biorecognition molecules are attached to the nitrocellulose membrane in the indicator region 15. Downstream of the indicator region 15, the width of the nitrocellulose membrane first increases at 14c to equal the width of the substrate 10 and then extends along the substrate 10 for a short distance.

Overlapping the membrane 14 at the downstream end of the strip (the right-hand end in Figure 3), is an absorption pad 16. The volume of the absorption pad 16 may be increased to provide the largest possible volume for drawing liquids through the membrane. Alternatively, the volume of the absorption pad 16 may be arranged so that a predetermined volume of the sample will be drawn through the indicator region 15 and into the absorption pad 16.

The assay strip is manufactured by first laminating together a substrate 10 and a nitrocellulose membrane 11 covering the part of the upper surface of the substrate. The conjugation pad 13, sample pad 12 and absorption pad 16 are then also laminated onto the substrate 10, with the absorption pad 16 and conjugation pad 13 overlapping the membrane 11, and the sample pad 12 overlapping the conjugation pad 13.

The nitrocellulose membrane 11 is then cut or ablated using a laser to cut down through the nitrocellulose membrane 11 to the substrate 10 along lines defining the tapered edges 14a , the side edges 14b and the tapered edges 14c. The laser used in the cutting process may be a 40 W laser operated at 35 to 45% power, with a focused spot size of about 150 µm, delivering pulses at 1000 DPI resolution (i.e. 25.4 µm spacing). The operating frequency, pulse length and pulse energy are adjusted so that as the laser is moved in a raster pattern, sufficient energy is delivered at each relevant point on the membrane to cut through to the underlying substrate.

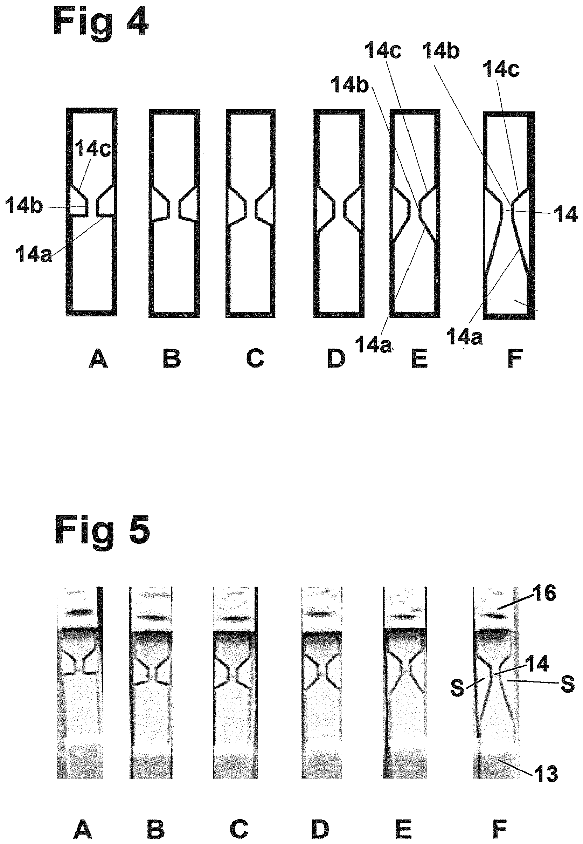

Figures 4A to 4F are schematic diagrams of different assay strips incorporating the micro-fluidic concentrator of the first aspect of the invention. Figures 5A to 5F are corresponding photographs of assay strips, with membranes corresponding respectively to the schematic diagrams of Figures 4A to 4F, and showing the absorption pad 16 and conjugation pad 13.

Figures 4A to 4F show different configurations of the laser cutting lines which form the edges 14a, 14b and 14c which define the effective edge of the nitrocellulose membrane and contain the analyte and reagents within this part of the nitrocellulose membrane. As can be seen in the corresponding Figures 5A to 5F, is not necessary to remove the surplus portions S of the nitrocellulose membrane, provided the width of the cut [kerf] made by the laser cutting or ablation process is greater than about 0.5mm, and possibly up to 1 mm. The cut must be wide enough to prevent liquid passing across the cut by capillary action, so that liquid is constrained to flow through the third zone. The cut must extend down through the nitrocellulose membrane as far as the substrate 10, and may extend into the substrate 10. In some embodiments, the cut may extend through the substrate 10 and a region S of the membrane and the corresponding part of the substrate may be removed.

Although nitrocellulose membranes are widely used in the manufacture of lateral flow membranes, the present invention is applicable to numerous other combinations of transport membrane and backing. Among those, paper formed with a paraffin film laminated backing constitutes an alternative to conventional paper fluidics, and delivers the benefit of highly defined and well supported (backed) layouts without the need of baking and masking the paper.

As can be seen in Figures 4A to 4F, the edges 14a may be angled either perpendicularly to the edges of the strip as seen in Figure 4A, or may converge at different angles to the edge of the strip as seen in Figures 4B to 4F. By tapering the edges at different angles, the rate of flow of the analyte through the assay device can be controlled. For example, by using a more acute angle, as shown in Figure 4F the flow rate is slowed gradually as the liquid flows through the absorption pad 13 to collect biolabels and then approaches the narrow test zone. This causes the liquids to flow slowly through the test zone, allowing greater time for the biomarked analytes to attach to the biorecognition molecules in the test zone. The sensitivity of the test is thus improved, in that the captured labelled analytes are concentrated in a smaller region of the nitrocellulose membrane, making visual detection easier due to the denser colouration of the indicator region 15. Depending on the analyte to be detected, and the biolabels and biorecognition molecules used, and appropriate degree of convergence of the edges 14a may be selected. In the example shown in Figure 4F, the edges 14a are angled such that the membrane tapers from a width of 5 mm to a width of 1 mm, over a distance of about 5 mm along the length of the strip. In the example of Figure 4A, the edges 14a are angled perpendicularly to the edges of the strip, and in the example of Figure 4B the edges 14a are angled such that the membrane tapers from a width of 5 mm to a width of 1 mm, over a distance of about 0 5 mm.

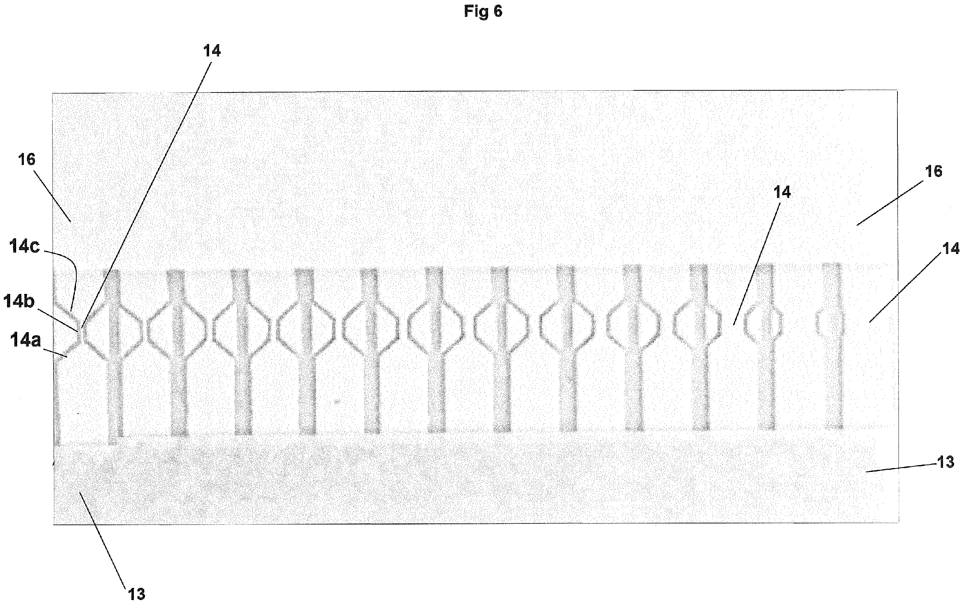

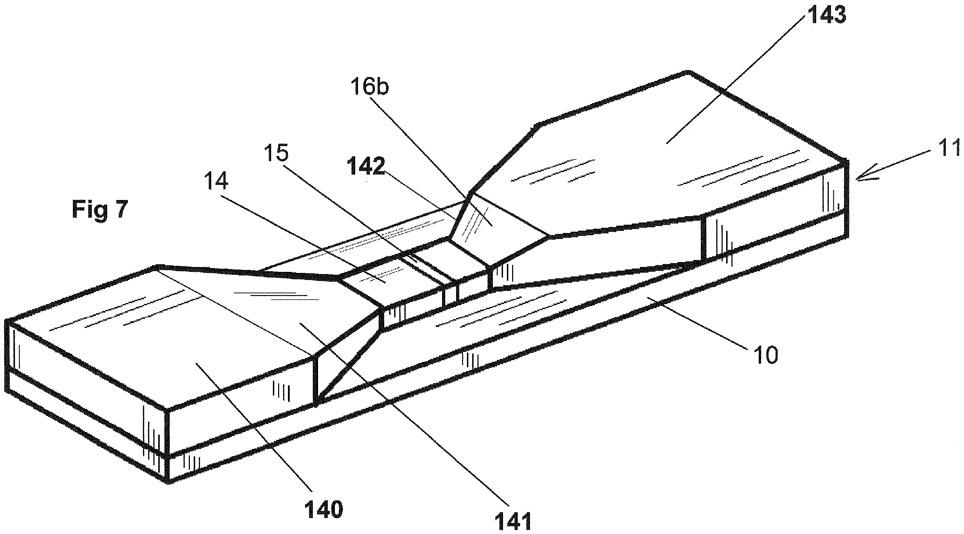

In the example shown in Figures 6 and 7, the width of the test zone 14 is from about 0.5mm to 1.5mm, and is preferably about 1 mm. The length of the test zone 14 may be from 1 to 2 mm in the flow direction. Within this test zone, the indicator region is formed by depositing a solution of the biorecognition molecules and drying the strip to fix the biorecognition molecules to the nitrocellulose membrane. The indicator region may extend over all or part of the length of the test zone 14 in the flow direction. Preferably the indicator region extends over a predetermined part of the length of the test zone 14.

Figure 6 is a view similar to figure 5, and illustrates the cutting of the nitrocellulose membrane in a plurality of test strips, prior to their separation into individual strips. An absorption pad 16 extends across the upper ends of all of the individual membranes 14, and a conjugation strip 13 extends across the lower ends of the membranes (as seen in the Figure. The test strip illustrated at the right-hand end of the Figure has at its lower end the conjugation pad 13, then the membrane 14 and at its upper end the absorption pad 16. The edges of the membrane have been cut to only slightly reduce the width of the membrane 14. The strip at the left-hand and likewise has a conjugation pad 13 the membrane, and an absorption pad 16. The membrane has been cut to form the edges 14a, 14b and 14c to produce a narrow third zone 14. Between these two extremes,, different configurations of cuts are illustrated to show different widths of the test zone 14 which may be formed in the test strip by laser ablation along the lines 14a, 14b and 14c in each strip. In this embodiment, the width of the test zone varies from about 0.5 mm in the left-hand strip to about 4.5 mm in the right-hand strip.

In order further to concentrate the indicator region 15 in which analytes are bound, the nitrocellulose membrane 11 may be reduced not only in width as shown in Figure 3, but it may also be reduced in depth as shown in the membrane illustrated in Figure 7.

In the membrane illustrated in Figure 7, the upstream end region 140 (the left-hand end as seen in the Figure) is of the same width as the substrate 10. Adjacent to this end region 140 is a tapering region 141 which is not only reduced in width, but is also gradually cut away or eroded in height (thickness) in the downstream direction, to reduce the height of the nitrocellulose membrane possibly to between 10% and 90%, or more preferably 20% to 50% of the full height (thickness) of the membrane.

The test zone 14 with its indicator region 15 is of substantially constant width and height, and leads to an expansion region 142 and then to an end region 143. The expansion region 142 is eroded to form an inclined face 16b to connect the upper face of the test zone 14 to the upper face of the end zone 143. In the embodiment shown, the inclined face 16b is angled so that the nitrocellulose membrane of the fourth zone 16 reaches its full thickness before reaching the full width of the strip. The inclined face 16b may alternatively be set at a different angle, or may be perpendicular to the plane of the substrate 10. The cross-sectional area of the membrane thus reduces, in the flow direction, at the tapering region 141 and remains substantially constant through the test zone 14 after which it initially rapidly increases as the liquid passes beneath the inclined face 16b of the expansion region 142 and then increases less rapidly, finally becoming constant through the downstream end region 143. The effect of this variation in cross-section is that the sample placed in the first zone 12 is first absorbed, and a "leading surface" of the liquid then moves along the membrane in the flow direction. As the leading surface moves through the second zone 13, its speed of advance reduces because of the reduced cross-sectional area of the membrane the sample is mixed with bio labels in the second zone 13, these biolabels becoming attached to the analyte molecules. By slowing down the rate of advance of the leading surface during its passage through this zone, the sample remains in contact with the bio labels for a longer time to promote their attachment to the analyte. The leading surface then passes at a substantially constant rate through the third zone 14, drawing the sample at a substantially constant initial flow rate through the test zone. As the leading surface then moves into the fourth zone, its speed of advance is increased by the increasing cross-sectional area of the membrane, so that the mixed bio labelled sample is quickly drawn through the test zone.

In the current invention, the technique of CO₂ laser ablation is used not only to define 2D layouts by removing the nitrocellulose membrane along the edges 13a, 14a and 16a without damaging the plastic backing substrate 10, but also to allow 3D structuring by controlling the intensity and the movement of the laser to locally reduce the thickness of the membrane. Typically the laser is applied at a resolution of 1000 PPI, and by using a 40 W laser to deliver a pulse every 5 msec, the entire depth of the membrane is cut through to produce the cut edges 13a, 14a and 16a without damaging the underlying substrate 10.

To produce the inclined upper surfaces such as the inclined face 16b, the laser may be raster-scanned across the membrane at a reduced power level, to produce different amounts of ablation of the thickness of the membrane. For example the laser beam may be set at constant reduced power, and be passed more often over regions where a greater depth of membrane is to be removed. The skilled man will easily determine combinations of laser power and scanning speed which can achieve the required sculpting of the membrane in three dimensions.

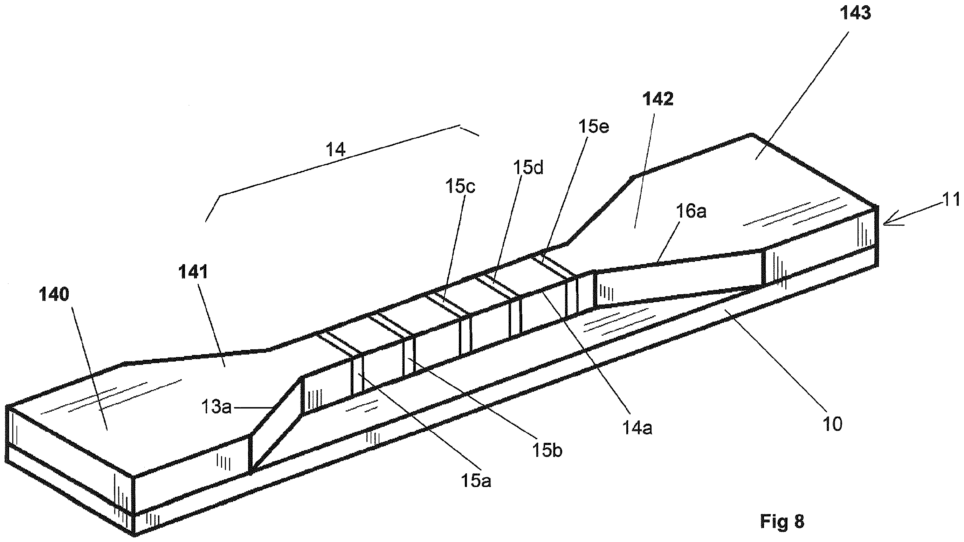

An assay including multiple test zones which can give a clear quantitative response by visual inspection is also contemplated in this aspect of the invention. Such a device is illustrated schematically in Figure 8, using the same reference numerals for corresponding parts as were used in Figure 3.

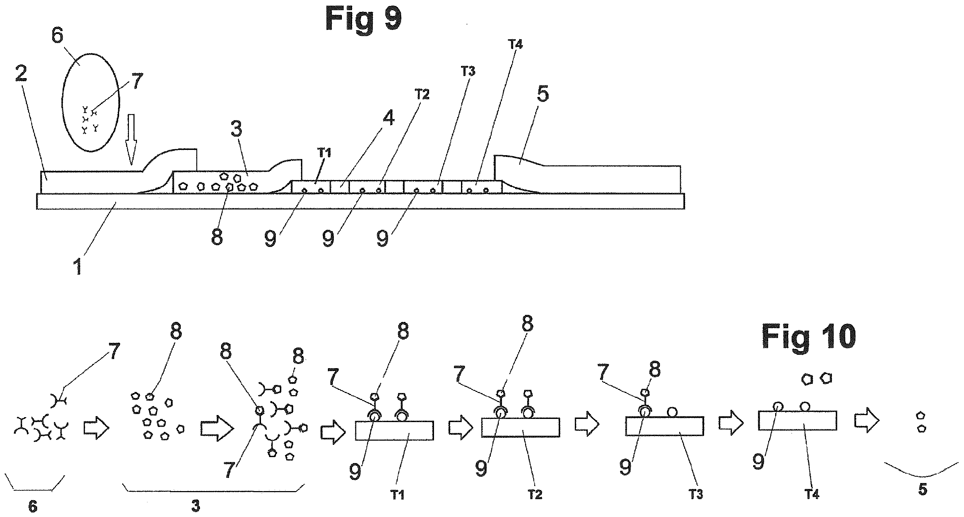

In Figure 8, there is shown a nitrocellulose membrane 11 for a test strip. The membrane is attached to, and supported by, a substrate 10. A sample pad (not shown) and a conjugation pad (not shown) will be provided at the upstream end of the membrane, and an absorption pad (not shown) will be arranged on the membrane at the downstream end of the membrane. At the upstream end of the strip (the left-hand end as seen in Figure 10), the nitrocellulose layer 11 has an upstream end region 140 of the same width as the substrate 10. Adjacent to this end region 140 is a tapering region 141 in which the width of the nitrocellulose membrane reduces.

Contiguous with the tapering region 141 is a test zone 14 which includes a number of indicator zones 15a to 15e spaced along the length of the test zone 14. At each indicator zone, a predetermined quantity of biorecognition molecules is immobilised on the nitrocellulose membrane, as will be described in more detail later.

Contiguous with the downstream end of the test zone 14 is an expansion region 142, in which the width of the membrane increases. Contiguous with the expansion region 142 is an end region of constant cross-section 143. The downstream end of the membrane is in contact with an absorption pad (not shown) whose volume, and thus the volume of liquid it can accommodate, is such as to ensure that a sufficient quantity of the analyte sample is drawn through the strip by capillary action.

The membrane of Figure 8 functions in the manner similar to the membrane described above in relation to the strip of Figure 3, except in the membrane of Figure 8 the test zone 14 comprises five indicator zones 15a to 15e, rather than the single indicator zone 15 shown in Figure 3.

Figure 9 and Figure 10 are schematic illustrations similar to Figures 1 and 2 of a test strip having multiple indicator zones. Figure 9 illustrates a side view, while Figure 10 schematically illustrates the operation of the test strip. The reference numbers used in Figures 9 and 10 correspond to those used in Figures 1 and 2.

Referring now to Figures 9 and 10, the sample pad 2 collects the sample fluid 6 which contains a number of the target analytes 7. In this example, the sample fluid 6 represents the amount of fluid which can be absorbed by the absorption pad 5. The actual amount of the sample applied to the sample pad 2 may be many times this amount, but the capacity of the absorption pad 5 is arranged so that only this amount of fluid will be drawn through test zone T4 and into the absorption pad 5.

The collected fluid is then drawn by capillary action into the conjugation pad 3, which is pre-loaded with secondary biolabels 8 specific for the target analytes 7 in the sample. The biolabels 8 attach to the target analytes 7 in the conjugation pad 3.

The mixture is then drawn through the nitrocellulose membrane 4, which includes a first test zone T1 in which a predetermined number of biorecognition molecules 9 are attached to the porous membrane. In each test zone T1 to T4, only two biorecognition molecules 9 are shown, in order to illustrate the effect. In test zone T1, some (in this case two) of the biolabelled target analytes 7 also attach to the biorecognition molecules 9, capturing some of the target analytes 7 in this test zone T1. If there is a high concentration of analytes 7 in the sample, and thus a high number of analytes 7 with biolabels 8 attached to them, then the biorecognition molecules 9 in test zone T1 will be unable to capture all of the labelled analytes 7.

The sample liquid, still containing uncaptured labelled analytes 7, then passes through the membrane 4 to a second test zone T2, in which a further predetermined number of biorecognition molecules 9 are attached to the membrane 4. Once again, some of the biolabelled analytes are captured by the biorecognition molecules 9. The sample then passes on to a third test zone T3 where further labelled analytes 7 are captured by biorecognition molecules 9. The sample then finally passes to a fourth test zone with a further predetermined number of biorecognition molecules 9, and finally the excess sample fluid is collected by the adsorption pad 5.

In each of the test zones T1 to T4, a predetermined number of biorecognition molecules 9 is immobilised on the nitrocellulose and each zone thus captures a predetermined amount of the biolabelled analyte from the sample fluid. The volume of fluid which is drawn through the device and into the absorption pad 5 is known, and thus the amount of the sample which has passed through each of the test zones is also known. Clearly, the leading part of the sample passes through all of the test zones T1 to T4 on its way to the absorption pad 5, while the part of the sample which is last to leave the sample pad 2 may pass only through some or none of the test zones T1 to T4.

Since each test zone captures a predetermined amount of labelled analyte from the sample fluid, then the number of test zones which have captured sufficient analyte to give a positive reading indicates the amount of analyte in the fluid sample flowing through the test zones.

In the simplified example illustrated in Figure 10, the initial fluid sample includes five analyte molecules 7, and each test zone can capture two analyte molecules. In the conjugation pad 3, bio-labels 8 attach to the five analyte molecules. In test zone T1, two of the bio-labelled analyte molecules are captured, and thus three pass on to test zone T2. In test zone T2, a further two of the bio-labelled molecules are captured, and the sample then passes on to test zone T3. In test zone T3, the remaining bio-labelled analyte molecule is captured, and the remainder of the sample then passes on through test zone T4 where no further analyte is captured, and into the adsorption pad 5.

In test zones T1 and T2 both of the biorecognition molecules 9 successfully capture a bio-labelled analyte molecule, and thus a clear positive result is indicated at test zones T1 and T2. Test zone T3 captures only one of bio-labelled molecules and thus may give a faint or inconclusive result. Test zone T4 captures no labelled molecules and thus gives a negative result. Thus, a visual inspection of the test strip will show the first and second test zones as returning a positive result, and a faint result at the third test zone. Since the user is aware of the volume of the initial sample of fluid which has passed through the test zones, and the number of molecules captured at each test zone, then a quantitative indication of the concentration of analyte in the initial sample is given by the number of test zones returning positive results.

To assist the user, the test strip may be printed with indicia showing the concentration level of analyte in a sample which corresponds to the number of test zones returning a positive result. For example, "0.1%" may be marked on the test strip adjacent test zone T1, "0.2%" may be marked on the test strip adjacent test zone T2, and so on. The user can simply read off the concentration against the last test zone in the series which returns a positive result.

Referring now to Figure 8, the indicator zones 15a, 15b etc correspond to the test zones T1, T2 etc of the schematic strip described in relation to Figures 9 and 10. A sample liquid placed in the sample pad of the strip will collect biolabels on the relevant analytes in the conjugation pad, and will then pass, by capillary action, sequentially through the indicator zones 15a to 15e to be collected in the absorption pad

The user simply looks at the indicator zones 15a to 15e, counts how many indicator zones have produced a positive result, and determines the concentration of analyte in the sample on the basis of this number. Alternatively, the user may locate the last indicator zone in the flow direction to produce a positive result, and may read off from the test strip a concentration figure marked on the strip adjacent that indicator zone. The narrowed shaping of the test zone 14 concentrates the bio labels in small regions of the membrane and thus the colour change produced by the capture of bio labelled molecules is clearly visible.

The test strip illustrated in Figure 8 has a linear test zone 14 with five indicator zones 15a to 15e. To deposit the biorecognition molecules in the indicator zones thus requires repeated passes with a depositor head, or simultaneous passes with multiple depositor heads, and this can result in a lack of uniformity in the deposition of the biorecognition material.

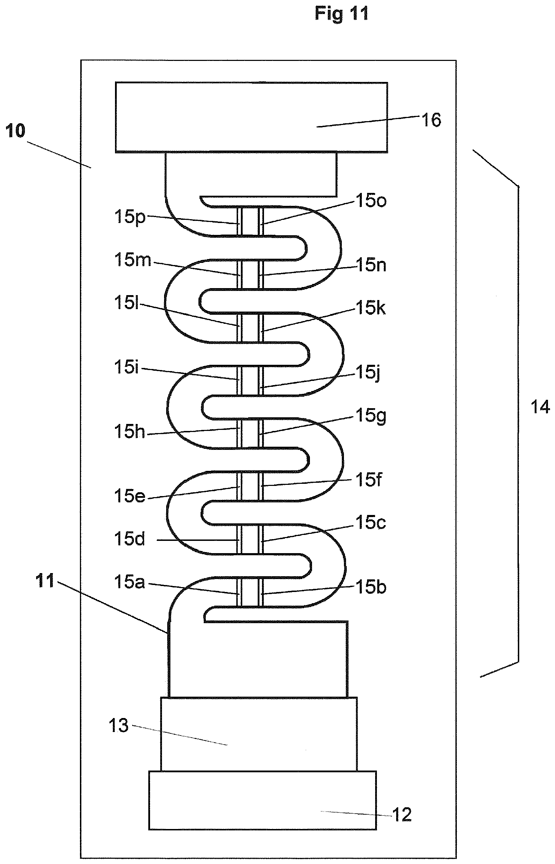

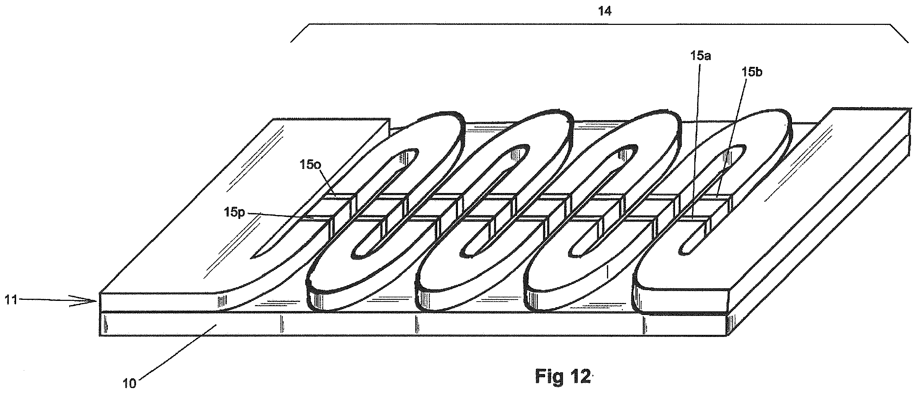

In an alternative embodiment, illustrated in Figures 11 and 12 the precision cutting ability of the laser is used to produce a test strip which has a larger number of indicator zones 15 but which enables the indicator zones 15 to be deposited in a single pass by a single depositing head and thus guarantee uniformity of the deposition of material in each of the zones.

In Figure 11 there is shown a substrate 10 on which is arranged a nitrocellulose membrane 11 for a test strip. On the substrate 10 adjacent the lower end of the strip (as seen in the Figure) is a conjugation pad 13, and next to that a sample pad 12 for receiving a liquid sample. The conjugation pad 13 is provided with biolabel molecules. The membrane 11 provides a serpentine path from the conjugation pad 13 to the fourth zone 16.

The assay strip is formed by arranging nitrocellulose membrane on the substrate, the membrane being overlapped at its respective ends by the absorption pad and the conjugation pad. The sample pad is then applied, to overlap the conjugation pad. Continuous lines of biorecognition material are then deposited, in this case two lines extending in the length direction of the membrane. The membrane is then cut and shaped to form the serpentine path, the serpentine path repeatedly crossing the lines of deposited biorecognition material.

The serpentine path is formed by laser cutting or ablation of the central part of the nitrocellulose membrane of the test strip when the membrane 11 is mounted to the substrate 10. The regions of the membrane outside the serpentine path may be entirely removed, or may be left in place but separated from the serpentine path by a cut of sufficient width to prevent fluid from leaving the serpentine path. Because the serpentine flow path is formed after the biorecognition material has been deposited and dried, there is no risk that additional biorecognition material may reabsorb into the flow path or the indicator zones. If the regions of the membrane outside the flow path are entirely removed or ablated, an accurate and uniformed the position of biorecognition material at each indicator zone may be achieved.

Indicator zones 15a to 15p are formed at the intersections of the serpentine path and the lines of biorecognition material. A fluid sample deposited on the sample pad 12 will pass through the conjugation pad 13 by capillary action and the analyte molecules will collect respective bio labels. The labelled sample then passes sequentially through the indicator zones 15a to 15p until finally reaching the adsorption pad 16 of the test strip.

At each indicator zone 15a to 15p, a predetermined quantity of biorecognition molecules is deposited. Each indicator zone may have substantially the same number of biorecognition molecules, deposited for example by placing a known quantity of solution containing the biorecognition molecules at each indicator zone and drying the liquid to deposit the molecules on the nitrocellulose membrane. Alternatively, the indicator zones may have different numbers of biorecognition molecules. This can be arranged, for example, by depositing a line of solution containing biorecognition molecules, and adjusting the width of the flow path at each indicator zone. The path may be made wider if more recognition molecules are required at a particular test zone, or narrower if fewer are required. In the illustrated embodiment of figures 11 and 12, the indicator zones 15a, 15d, 15b, 15h, 15i, 151, 15m and 15p are arranged in a first straight line, and the indicator zones 15b, 15c, 15f, 15g, 15j, 15k, 15n and 15o are arranged in a second straight line. The biorecognition molecules may thus be placed into the membrane by a depositor making a single straight pass along the length of the strip and depositing an amount of a solution containing the biorecognition molecules each time it passes over the serpentine path.

In the embodiment shown, each straight section of the serpentine path includes two indicator zones. Indicator zones may additionally or alternatively be positioned on the curved parts of the serpentine path, and more or fewer than two indicator zones may be positioned in the straight sections of the serpentine path.

In the embodiment shown, the serpentine path includes straight sections arranged transversely of the length of the strip. It is foreseen that in alternative embodiments, the serpentine path may have its straight sections extending along the length of the strip.

Figure 12 is a perspective view of the nitrocellulose membrane 11 of Figure 11, mounted to a substrate 10. In the illustrated embodiment, the serpentine path has a substantially constant width. It is, however, foreseen that the path may be narrowed at the indicator zones, in order to concentrate the capture of biomarked reagents into a smaller area to facilitate visual reading of the device, for example by making a colour change more intense.

In use, a sample of fluid will be placed in the sample pad 12, and will pass through the conjugation pad 13 to collect biolabels and then pass along the serpentine path to fill the fourth zone 16 by capillary action. A predetermined amount of the sample will have passed through each of the indicator zones, the sample passing sequentially through zones 15a to 15p. The user then either counts how many indicator zones have returned a positive result and determines the analyte concentration from that number, or simply looks for the most downstream indicator zone returning a positive result and reads off a concentration value printed adjacent to that test zone.

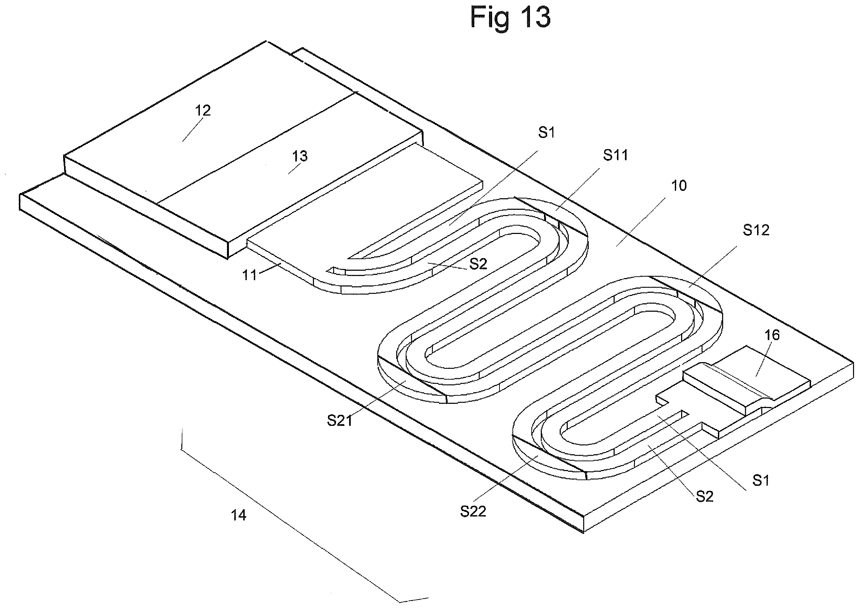

Figures 13 is a perspective view of a further embodiment of the assay device. Like numbers are used to designate parts corresponding with the embodiment of Figure 12. The capillary membrane 11 of Figure 13 is mounted on a substrate 10 and has at one end a rectangular area which is in contact with a conjugation pad 13, which in turn is in contact with a sample pad 12 for receiving a liquid sample. The conjugation pad 13 includes biolabel molecules for mixing with the liquid sample. At the other end of the strip there is a rectangular adsorption pad 16 into which the sample is drawn by capillary action. Connecting the conjugation pad 13 to the rectangular adsorption pad 16 is a central section which forms the third zone 14 of the test strip, and provides a pair of side-by side serpentine paths S1 and S2 extending from the conjugation pad 13 to a rectangular terminal area, overlapped by the absorption pad 16.

The membrane of Figure 13 thus provides two separate serpentine paths, which will each receive a portion of the sample placed into the first zone 12, after it has passed through the conjugation pad 13 and collected biolabels. The conjugation pad 13 may include one or more different types of biolabels, specific to different analyte molecules.

The serpentine path S1 has two indicator zones S11 and S12, positioned on the curved parts of the path. Likewise, the serpentine path S2 has two indicator zones S21 and S22, positioned on the curved parts of the path. Each path may have a larger number of curved regions, and a correspondingly larger number of indicator zones. The indicator zones on each path are provided on the larger-radius curves of the serpentine path, and these may be positioned in a line such that a depositor may move in a linear direction along the length of the strip and pass over each of the indicator zones S11 and S12 of one of the paths to place a quantity of solution containing the biorecognition molecules at each indicator zone. The same or a different depositor may make a linear movement along the length of the strip to place a quantity of solution containing the biorecognition molecules at each indicator zone S21 and S22 of the other path. These deposition operations are performed before the membrane 11 is formed into its final shape by laser ablation of the unwanted material of the membrane 11 to leave the serpentine paths. The laser ablation process may remove any parts of the absorption pad, conjugation pad and/or sample pad on which biorecognition material has been deposited, or the absorption pad, conjugation pad and sample pad may be pre-shaped so that they do not extend into the regions where the biorecognition material will be deposited.

As has been described in relation to the previous embodiments, the sample liquid is drawn along the paths S1 and S2, and biorecognition molecules at the indicator zones S11, S12, S21 and S22 capture labelled analyte molecules from the sample. The indicator zones on one of the paths may each capture more analyte molecules than respective indicator zones on the other path, so that by counting the number of indicator zones which return a positive result, the concentration of analyte in the sample can be determined. For example, one of the paths may have five indicator zones which give indications of the concentration of analytes between zero and 10%, while the other path has five indicator zones which give indications of the concentration of analyte between 10% and 20%.

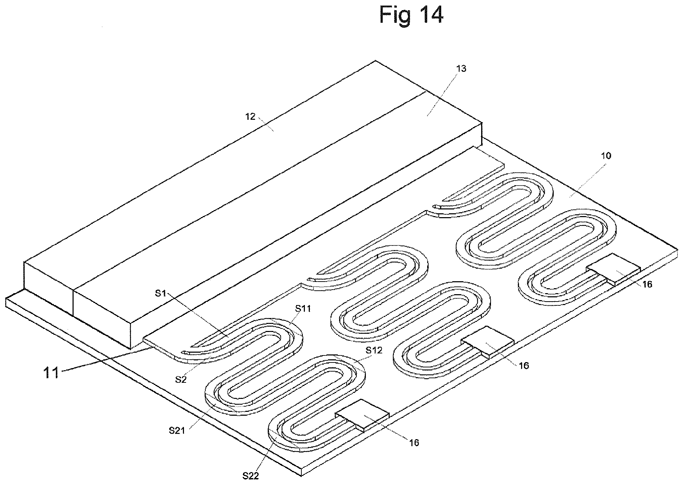

Figure 14 illustrates an alternative embodiment of the membrane of Figure 13, in which three pairs of serpentine paths lead from a rectangular area of membrane 11 at one end of an assay strip to three respective absorption pads 16. The rectangular area is in contact with a conjugation pad 13, which is in turn in contact with a sample pad 12 onto which the sample is placed, so that the sample may pass by capillary action through the sample pad 12, through the conjugation pad 13 and into the membrane. The conjugation pad 13 includes biolabels selected so that they attached to the analytes of interest in the sample as it passes through the conjugation pad 13 and into the adjacent rectangular area of the membrane.

Indicator zones S11, S12, S21 and S22 include biorecognition molecules which capture the labelled analyte molecules. The biorecognition molecules of the indicator zones of all six paths may be selected to detect the same analyte, and may be provided in different numbers in each of the strips in order to provide indications of different ranges of concentration of analyte in the sample. Alternatively, the biorecognition molecules in each of the three pairs of paths may be selected so as to detect different analyte molecules in the sample, and the two paths of each pair may have different numbers of biorecognition molecules in each indicator zone to provide an indication as to whether the concentration of the respective analyte molecules is within one or other of two ranges, as described earlier.

Each pair of paths terminates in a respective pad 16. By adjusting the sizes of the pads 16, the amounts of the sample which pass along each pair of pathways can be controlled. For example, if all three of the pads 16 are of the same size, then the same volume of sample will pass along each of the pairs of paths in order to fill the pads 16. If one of the pads 16 is larger than the others, then a larger amount of the sample will pass along the pair of paths connecting to that pad, drawn by capillary action until the pad 16 is filled.

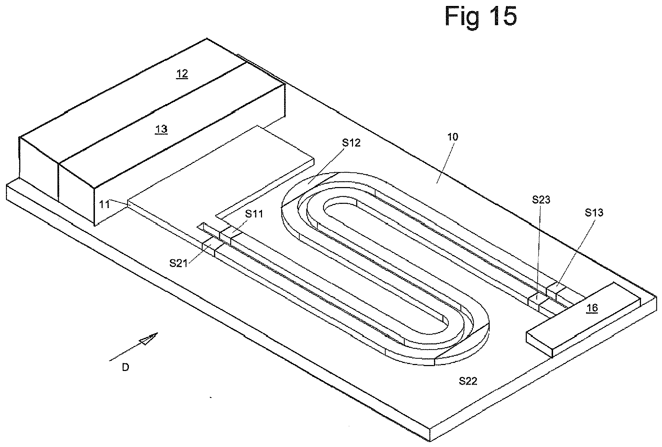

Figure 15 illustrates an alternative embodiment to the embodiment of Figure 13, but in this case the serpentine paths are arranged so that their straight sections extend along the length of the strip rather than across the width of the strip. The reference numerals correspond to those used in Figure 13 for like components.

In the membrane illustrated in Figure 15, a rectangular area at one end of the membrane is in contact with a conjugation pad 13 which in turn is in contact with a sample pad 12. Leading from the rectangular area are two serpentine paths S1 and S2, each of which has three indicator zones S11, S12 and S13 in path S1, and S21, S22 and S23 in path S2. The indicator zones may be formed by depositing a solution of the biorecognition molecules from a depositor head moving in the transverse direction of the strip (in the direction of arrow D in Figure 15), for example so as to deposit the solution sequentially at indicator zones S21, S11 and S12. The depositor head may make a subsequent deposition run in the opposite direction to deposit the indicator zones S13, S23 and S22. Alternatively, to depositor head is made simultaneously move across the strip in the direction of arrow D to deposit all of the biorecognition material in a single operation.

Deposition of the biorecognition material is preferably carried out before the membrane 11 is sculpted to form the serpentine pathways, by depositing linear zones of biorecognition material and ablating the membrane to form the pathways such that they cross the linear zones.

A sample is placed in the sample pad 12 and passes through the conjugation pad 13 where there are located biolabel molecules selected to attach to the analyte molecules in the sample. The sample then passes through the rectangular area and into the two serpentine paths S1 and S2. The portion of the sample passing along the serpentine path S1 passes sequentially through the three indicator zones S11, S12 and S13, and finally into the pad 16. Likewise, the portion of the sample passing along the serpentine path S2 passes sequentially through the three indicator zones S21, S22 and S23, and finally into the pad 16. As described in relation to figure 8 above, the concentration of analyte present in the sample may be determined by inspecting the indicator zones in each pathway, and either counting the zones which give a positive indication, or determining the most downstream zone in each pathway which gives a positive indication.

While a single pad 16 is shown in the illustrated embodiment, each serpentine path S1, S2 may have an individual respective pad 16, and these individual pads may be of the same or different capacities to regulate the amount of the labelled sample which is drawn along each of the two paths.

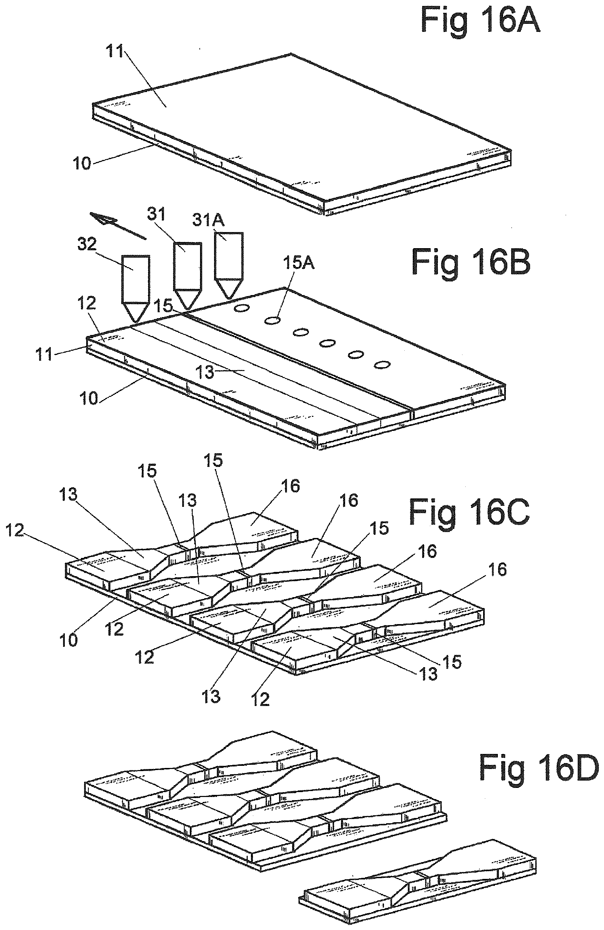

Figures 16A to 16D illustrate the steps in a process for producing the shaped membranes. Like reference numerals have been used to correspond to the references used in earlier figures. The process starts with a sheet of capillary membrane 11 bonded to a hydrophobic backing sheet 10, as seen in Figure 16A. The sheet is preferably an elongated sheet, whose width corresponds to the lengths of the individual assay strips to be formed. The membrane may be of nitrocellulose, or any other suitable porous material through which the sample will be drawn by capillary action. The backing sheet 10 may extend beyond the edges of the membrane, and may support an absorption pad at one side of the membrane, and conjugation pad and a sample pad at the other side of the membrane (not shown). The conjugation pad may be pre-treated with bio label molecules before it is applied to the substrate 10 to overlap the membrane 11.

The sheet is passed lengthwise under a depositor head 31, or the head 31 may be moved along the length of the sheet, in order to deposit a line of a solution containing biorecognition molecules at a predetermined transverse position on the sheet of capillary membrane. The solution is absorbed into the membrane and allowed to dry so that the biorecognition molecules become fixed in the capillary membrane in a linear region 15 extending along the sheet. More than one depositor head 31 may be used, or the depositor head 31 may be passed over the sheet multiple times, to produce a number of parallel regions 15 extending along the sheet, in order to form assay strips having multiple test zones.

Alternatively or in addition to the linear region 15, the solution of biorecognition molecules may be deposited as a series of discrete areas 15A in a linear array, from a depositor 31A.

Simultaneously, or as a subsequent deposition step, the biolabel molecules may be deposited as a solution from a second depositor head 32, to form a linear region 13 along the length of the sheet which will correspond to the conjugation pad where analytes from the sample will combine with the bio label molecules. The solution is left to dry so that the bio label molecules are then held in the membrane in the linear region 13. Likewise, a plurality of depositors may be used to deposit a plurality of different bio labels into the region 13. An untreated region may be left between the edge of the sheet and the region 13, to serve as a sample input region 12 in the finished assay strips. Alternatively, a separate sample pad and conjugation pad may be provided in fluid communication with the membrane. The biolabel molecules may be deposited onto the conjugation pad as a solution from the second depositor head 32, either simultaneously or sequentially with the deposition of the biorecognition molecules, and left to dry to deposit the bio recognition molecules in the conjugation pad.

The sheet of capillary membrane is then shaped into individual assay strips by a process which cuts and shapes the membrane 11 but leaves the substrate 10 intact. Preferably, the cutting and shaping process is performed by a laser which ablates the capillary membrane to remove unwanted areas of the membrane. The membrane may also be shaped by conventional cutting processes which cut through the membrane 11 but leave the substrate 10 intact. The cutting of the membrane is arranged such that the test area in each individual strip of membrane corresponds to a part of the region 15, or to a region 15A, of the membrane in which the biorecognition molecules are fixed, and leads from the linear region 13 or the conjugation pad where the bio labelled molecules are fixed in the membrane to the absorption pad 16.

Individual assay strips are prepared by separating the shaped strips, by cutting the substrate 10 between adjacent pairs of shaped strips, so that each individual assay strip comprises a part of the membrane 11, and optionally also parts of the conjugation pad, the sample pad and the absorption pad. The substrate 10 may be cut completely through, or may be formed with a line of weakness or frangible region allowing individual strips to be torn from the sheet.

The micro-fluidic pattern in the nitrocellulose membrane could control the sequence of addition of different reagents by design the fluidic path or creating flow delay micro-pattern. This approach could also be useful for example for silver enhancement of gold nanoparticles (AuNP), and/or enzymatic biolabel systems, which the sequential addition of second reagent is necessary.

The technology has been described in relation to specific embodiments of assay devices, but is applicable to a wide range of affinity-based lateral flow tests including but not limited to tests for detecting and/or quantifying antibodies, peptides, ligands, cells, cell surface receptors, nucleic acids, synthetic mimetic nucleic acids, recombinant affinity molecules, synthetic receptors, molecular imprinted polymers; in combination of biolabel systems including but not limited to metal nanoparticle labels, inorganic nanoparticle labels, organic nanoparticle labels, carbon-based labels, quantum dot labels, luminescent labels, up-convention luminescent labels, chemiluminescent labels, enzyme labels, electrochemical active labels and redox active labels etc.

The assay strips of the present invention may be used with an associated reader device in order to obtain the signal output. Alternatively, the assay strip may be simply read by the user optically inspecting the test zone or zones.

In an alternative embodiment, the assay strips of the present invention may be used in conjunction with an electrical or electronic reader apparatus. In such an embodiment, the reader apparatus has an electrical connector with multiple contacts, and an array of electrodes is formed on assay strip in contact with the nitrocellulose membrane of the assay strip. The electrodes, and conductive leads joining them to a connector at the end of the assay strip, may be formed on the substrate before the nitrocellulose membrane is applied to it, and the sculpting of the membrane arranged so that the electrodes are positioned at the required points on the flow path. The array of electrodes formed on the assay strip preferably includes a pair of electrodes placed on opposite sides of each test zone, so that by selectively applying voltage or current to the electrodes, a current or voltage may be applied across each test zone. The reader apparatus includes a processor and a power source, and applies current or voltages between the electrodes of its array. Measuring the responses between corresponding pairs of electrodes on the assay strip allows the processor to calculate the test result, by measuring for example resistance or impedance of the membrane in the test zone. When the bio labels are electrically conductive such as gold nanoparticles, the capture of analyte and bio label particles in the test zone alters the electrical properties of the test zone, and thus the measured voltage, resistance or impedance may be compared to a predetermined value to determine whether the test has been positive or negative.

## Claims

1. A method of manufacturing an assay strip membrane, comprising the steps of:
laminating a sheet of porous membrane (11) to an impermeable substrate (10);
applying a solution of biorecognition molecules in a first linear region (15, 15A) extending across the sheet; and
forming a flow path in the assay strip membrane by shaping the porous membrane (11), the assay strip flow path including an indicator a test zone (15) formed from a part of the first linear region.

2. A method of manufacturing a plurality of assay strip membranes, comprising the steps of:
laminating a sheet of porous membrane to an impermeable substrate;
applying a solution of biorecognition molecules in a first linear region extending across the sheet;
forming the plurality of assay strips by shaping the porous membrane, each assay strip extending perpendicularly to the first linear region and including a flow path having a test an indicator zone formed from a part of the first linear region; and
cutting the substrate between each two adjacent shaped assay strips.

3. A method according to claim 1 or claim 2, wherein the solution of biorecognition molecules is deposited as a series of discrete areas (15A) in a linear array in the first linear region.

4. A method according to any of claims 1 to 3, further including the step of:
applying a solution of biolabel molecules in a second linear region extending across the sheet in parallel to the first linear region; and
forming the or each assay strip by shaping the porous membrane so that the or each assay strip includes a part of the second linear region.

5. A method according to any preceding claim, wherein the membrane is shaped such that the or each assay strip includes a serpentine flow path arranged to cross the first linear region at least three times such that at least three test zones are formed in the serpentine flow path.

6. A method according to any preceding claim wherein a plurality of parallel first linear regions containing biorecognition molecules are formed in the porous membrane, and wherein each assay strip includes flow path including a plurality of test zones each corresponding to a part of a respective oneeach of the first linear regions.

7. A method according to any preceding claim in which the step of forming the or each assay strip comprises removing portions of the porous membrane by a laser ablation process to define a flow path including a part of the first linear at region as a test zonleast one indicator zonee.

8. A method according to any preceding claim in which the step of forming the or each assay strip comprises removing portions of the porous membrane to define a flow path whose width and thickness vary along the length of the flow path.

9. An assay strip comprising a first zone for receiving a liquid sample containing analyte, a second zone containing biolabel molecules which are adapted to attach to analyte molecules in the sample, an indicator a test zone in which biorecognition molecules adapted to attach to analyte molecules are fixed, and a reservoir zone, wherein the arrangement being such that at least part of a liquid sample placed in the first zone is drawn along a flow path by capillary action through the second zone, and the indicator test zone and into the reservoir, and wherein the cross-sectional area of the assay stripflow path at the indicator test zone is made smaller than the cross-sectional area of the remainder adjacent regions of the stripflow path.

10. An assay strip according to claim 9, wherein the width and/or thickness of the capillary membrane at the indicator zone are reduced at the test zone of the flow path, compared to the remainder adjacent regions of the assay stripflow path.

11. An assay strip according to claim 9, wherein the cross-sectional area of the flow path in the capillary membrane varies between the first zone and the reservoir zone.

12. An assay strip comprising a first zone for receiving a liquid sample containing analyte, a second zone containing biolabel molecules which are adapted to attach to analyte molecules in the sample, a flow path and a reservoir zone, wherein the flow path provides fluid communication from the second zone to the reservoir zone, and wherein the flow path includes a plurality of indicator zones, each test zone comprisings at each of which are fixed a number of biorecognition molecules adapted to attach to analyte molecules, and a reservoir zone, wherein the arrangement being such that at least part of a liquid sample placed in the first zone is drawn by capillary action through the second zone, and sequentially through the indicator test zones and into the reservoir zone, and wherein the cross-sectional area of the flow path at the test zones is smaller than the cross-sectional area of end regions of the flow path.

13. An assay strip according to claim 12, wherein the indicator test zones are each configured to capture the same amount of target analyte.

14. An assay strip according to claim 12, wherein the indicator test zones are configured such that the amount of target analyte captured at each successive indicator test zone increases in the direction along the flow path.

15. An assay strip according to any of claims 12 to 14, wherein the membrane is shaped to form a serpentine flow path extending from the second zone to the reservoir, the plurality of indicator test zones being positioned in the serpentine flow path.
